Europäisches Patentamt

European Patent Office

Office européen des brevets

(19)

(11) Veröffentlichungsnummer : **0 389 583 B1**

# (12) EUROPÄISCHE PATENTSCHRIFT

(45) Veröffentlichungstag der Patentschrift :
**19.11.92 Patentblatt 92/47**

(51) Int. Cl.$^5$ : **A61K 35/28, A61K 45/05**

(21) Anmeldenummer : **89908965.0**

(22) Anmeldetag : **08.08.89**

(86) Internationale Anmeldenummer :
**PCT/DE89/00519**

(87) Internationale Veröffentlichungsnummer :
**WO 90/01326 22.02.90 Gazette 90/05**

(54) HERRICHTUNG UND VERWENDUNG EINES PHYSIOLOGISCH AKTIVEN POLYPEPTIDS ZUR AIDS-THERAPIE.

(30) Priorität : **12.08.88 DE 3827490**

(43) Veröffentlichungstag der Anmeldung :
**03.10.90 Patentblatt 90/40**

(45) Bekanntmachung des Hinweises auf die Patenterteilung :
**19.11.92 Patentblatt 92/47**

(84) Benannte Vertragsstaaten :
**AT CH DE FR GB IT LI NL**

(56) Entgegenhaltungen :
**DE-A- 3 151 979**
**Akt. Dermatol., vol. 10, 1984, Georg Thieme Verlag (Stuttgart, DE), R. Bauer et al., pages 174-175**

(56) Entgegenhaltungen :
**Science, vol. 235, 6 March 1987, C. Kolata, pages 1138-1139**
**Archives of Biochemistry and Biophysics, vol. 263, no. 2, June 1988, Academic Press Inc., M. Frangou-Lazaridis et al., pages 305-310**
**Chemical Abstracts, vol. 92, 1980 (Columbus, Ohio, US), M. Lenfant et al., p. 460, abstract no. 178859z, & Mol. Immunol. 1980, 17(1), 119-26**

(73) Patentinhaber : **HOR-FER-VIT PHARMA GMBH**
**H.-Brockmannstrasse 51**
**W-2900 Oldenburg (DE)**

(72) Erfinder : **KUHLMEY, Jürgen**
**Wehdestr. 6**
**W-2900 Oldenburg (DE)**

(74) Vertreter : **Goddar, Heinz J., Dr. et al**
**FORRESTER & BOEHMERT**
**Franz-Joseph-Strasse 38**
**W-8000 München 40 (DE)**

EP 0 389 583 B1

## Beschreibung

Die Erfindung betrifft die Verwendung eines physiologisch aktiven Polypeptids zur Herrichtung eines Arzneimittels zur Aidstherapie sowie dessen Verwendung in letzterer.

Das Aidsproblem, charakterisiert durch die Erkrankung einer mehr und mehr wachsenden Zahl von Menschen an vermutlich virusbedingt erworbener Schwäche des Immunsystems, harrt dringend einer Lösung. Alle bisher bekannten Behandlungsversuche haben sich im wesentlichen als erfolglos erwiesen. Weder ein Impfstoff noch ein wirkliches Heilmittel sind gefunden worden. Lediglich die in letzter Zeit zur Anwendung gekommene Retrovir-Therapie scheint, wenn schon keine Heilung, so doch eine Erhöhung der Überlebenschancen von Aids-Kranken mit sich zu bringen. Eine Wiederherstellung des Immunsystems in dem Sinne, daß die Aidserkrankung tatsächlich verschwinden würde, konnte bisher nicht erzielt werden.

Der Erfindung liegt die Aufgabe zugrunde, eine Arzneimittelspezialität zur Behandlug von Aidserkrankungen zur Verfügung zu stellen.

Erfindungsgemäß wird diese Aufgabe gelöst durch die Verwendung eines physiologisch aktiven Polypeptids, hergestellt durch vollständige enzymatische Proteolyse reticulo-endothelialreicher Organe von Säugetieren bei einer Temperatur von nicht mehr als 45°C, zur Herrichtung einer Arzneimittelspezialität zur Aids-Therapie.

Dabei kann vorgesehen sein, daß im Anschluß an die vollständige enzymatische Proteolyse das Reaktionsgemisch von den unlöslichen Bestandteilen getrennt und daraufhin das Polypeptid abgetrennt wird.

Bei der Erfindung kann auch so vorgegangen werden, daß im Anschluß an die vollständige enzymatische Proteolyse die Feststoffe mechanisch abgetrennt und die Proteolyse-Lösung eingeengt und lyophilisiert wird.

Die erfindungsgemäße Verwendung ist ferner vorzugsweise dadurch gekennzeichnet, daß die Proteolyse mit Papain, Pepsin oder Trypsin erfolgt.

Schließlich lehrt die Erfindung auch die Verwendung der hergerichteten Arzneimittelspezialität zur Aids-Therapie.

Der Erdindung liegt die überraschende Erkenntnis zugrunde, daß sich ein aus der DE-PS 16 17 566 und der DE-OS 31 51 979 bekanntes physiologisch aktives Peptid im Sinne einer weiteren Indikation zur Aids-Therapie eignet. Klinische Untersuchungen haben erwiesen, daß sich bei parentaraler oder oraler, vorzugsweise kombinierter Anwendung der Arzneimittelspezialität nach der Erfindung in 70% aller Behandlungsfälle, bei denen es sich unterschiedslos um Patienten mit vollklinischem Aids-Erscheinungsbild im Endstadium handelte, eine Vollremission erzielt werden konnte. Es besteht die begründete Aussicht, daß die Behandlungserfolge noch weitaus vollständiger sind, wenn nicht nur Patienten im Endstadium, sondern bereits in einem vorklinischen Aids-Stadium behandelt werden.

Zur näheren Erläuterung des Verfahrens zur Herstellung des erfindungsgemäß verwendeten und zur Herrichtung der Aids-Arzneitmittelspezialität verwendeten physiologisch aktiven Polypeptids wird auf die bereits genannten Druckschriften, nämlich DE-PS 16 17 566 und DE-OS 31 51 979, verwiesen.

In beiden Druckschriften ist im Sinne einer ersten Indikation die Verwendung des physiologisch aktiven Polypeptids zum Senken des Cholesterinspiegels im menschlichen Blut beschrieben.

Nachstehend wird die Erfindung anhand von Ausführungsbeispielen sowie unter Beschreibung einer klinischen Versuchsreihe im einzelnen erläutert. Es ist noch darauf hinzuweisen, daß sich Arzneimittelspezialitäten, bei denen das physiologisch aktive Polypeptid ausschließlich aus Milz gewonnen wurde, als besonders vorteilhaft erwiesen haben.

Beispiel 1:

5 kg frisch zerkleinerte Rindermilz wurden bei 40° C 24 Stunden mit 5 g Pepsin 1: 1000 der Proteolyse unterworfen, wobei 5 l destilliertes Wasser zugefügt wurden und der pH-Wert der Lösung mit Salzsäure auf 1,7 bis 1,9 eingestellt wurde. Daraufhin wurde das Reaktionsgemisch durch ein Papierfilter gefiltert. Zu der klargefilterten Lösung wurden 32 Gewichtsprozent Natriumchlorid gegeben. Der auf diese Weise erhaltene Niederschlag wurde abgetrennt und getrocknet und war für orale Anwendung geeignet, vorzugsweise in Form von Tabletten oder Dragees.

Beispiel 2:

5 kg Lymphdrüsen von Rindern wurden bei 40°C 72 Stunden mit 10 g Pepsin 1 : 1000 der Proteolyse unterworfen, wobei 5 l destilliertes Wasser zugefügt wurden und der pH-Wert der Lösung mit Salzsäure auf 1,7 eingestellt wurde. Daraufhin wurde das Reaktionsgemisch zentrifugiert. Zu der klaren Lösung wurden dann 20 bis 25 Gewichtsprozent Phenol gegeben. Die Phenolphase wurde von der Wasserphase im Scheidetrichter getrennt und das Phenol aus der Phenolphase durch Äther extrahiert. Das zurückbleibende Konzentrat, das das physiologisch aktive Polypeptid enthält, war an sich bereits in dieser Form für parenterale Verwendung geeignet. Zur weiteren Reinigung wurde jedoch das gesamte Konzentrat, das aus der Phenolphase durch Entfernen des Phenols gewonnen wurde, mit Wasser auf ein Trockengewicht von 10 % verdünnt. Durch Zugabe von 7,472 g $NaH_2PO_4$ und 0,784 g $Na_2HPO_4$ $2 H_2O$ wurde der pH-Wert dieser Mischung eingestellt, worauf der erhaltene Niederschlag durch Filtration oder Zentrifugieren entfernt wurde. Nach isotonischer Einstellung war das gereinigte Konzentrat zur parenteralen Anwendung geeignet.

Beispiel 3:

5 kg frisch zerkleinerte Schweinemilz wurden bei 45° C und einem pH-Wert von 8,0 6 Stunden lang durch 125 g Trypsin (1 g = 40 000 Fuld-Gross-Einheiten) abgebaut, wobei 5 l destilliertes Wasser zugefügt wurden und die Einstellung des pH-Wertes durch Natronlauge erfolgte. Die weitere Konzentrierung erfolgte wie im Beispiel 1.

Beispiel 4:

5 kg frisch zerkleinerte Rindermilz wurden bei 45° C 10 Stunden mit 40 g rohem Papain behandelt, wobei 5 l destilliertes Wasser zugefügt wurden und der pH-Wert der Lösung mit Natronlauge auf einen Wert von 7,0 eingestellt wurde. Die weitere Konzentrierung und Reinigung der Mischung erfolgte wie im Beispiel 2.

Beispiel 5:

5 kg Knochenmark von Rindern wurden bei 40° C 72 Stunden lang mit 10 g Pepsin der Proteolyse unterworfen, wobei 5 l destilliertes Wasser zugefügt wurden und der pH-Wert der Lösung mit Salzsäure auf 1,7 eingestellt wurde. Nach der Konzentrierung durch Phenolextraktion wie im Beispiel 2 wurde das Konzentrat durch Säulenchromatographie gereinigt.

Beispiel 6:

5 kg frisch zerkleinerte Rindermilz wurden bei 45°C 72 Stunden unter Zugabe von 10 g Pepsin 1 : 1000 der Proteolyse unterworfen, wobei 5 l Wasser zugeführt wurden und der pH-Wert der Lösung mit Salzsäure auf 1,7 - 1,9 eingestellt wurde. Anschließend wurde das Reaktionsgemisch durch einen Papierfilter gefiltert. Die klare Lösung wurde eingeengt und gefriergetrocknet. Das erhaltene Pulver eignet sich zur Herstellung von Tabletten oder Dragees.

Beispiel 7:

5 kg frisch zerkleinerte Lymphdrüsen von Rindern wurden bei 40°C 72 Stunden mit 10g Pepsin 1 : 1000 in 5 l Wasser aufgeschlämmt und 72 Stunden proteolysiert. Der pH-Wert der Lösung wurde zur Proteolyse mit Salzsäure auf 1,7 eingestellt. Nach Ende der Reaktionsdauer wurde das Reaktionsgemisch zentrifugiert und das Zentrifugat verworfen. Der Uberstand wurde bis zur Trockene eingeengt und liophilisiert. Der Rückstand ist das erfindungsgemäß verwendbare Polypeptid.

Beispiel 8:

Beispiel 7 wurde wiederholt, wobei die Lymphdrüsen von Rindern durch frisch zerkleinerte Schweinemilz ersetzt wurden.

Beispiel 9:

Die in Beispiel 6 beschriebenen Versuchsbedingungen wurden eingehalten, wobei anstatt Pepsin 1 : 1000 40 g rohes Papain verwandt wurde und der pH-Wert der Lösung mittels Natronlauge auf einen Wert von 7,0 eingestellt wurde.

Beispiel 10:

Die in Beispiel 6 geschilderten Versuchsbedingungen wurden wiederholt, wobei anstatt roher Rindermilz Knochenmark von Rindern eingesetzt wurde.

Beispiel 11:

Es wurde vorgegangen wie in Beispiel 1, wobei jedoch statt Rindermilz Schweinemilz verwendet wurde.

Beispiel 12:

Es wurde vorgegangen wie in Beispiel 2, wobei jedoch statt der Lymphdrüsen von Rindern Schweinemilz verwendet wurde.

Klinische Ergebnisse:

Mit nach Beispiel 11 hergestellten, enterisch beschichteten Tabletten (Dragees) und nach Beispiel 12 hergestellten bzw. befüllten Ampullen - beide Erzeugnisse sind unter der Bezeichnung POLYERGA® im Handel -, wobei die Ampullen also eine Glykopeptidlösung und die Tabletten die Peptidverbindung, jeweils auf Schweinemilzbasis gewonnen, enthielten, wurden zehn Aids-Patienten behandelt, bei denen bereits alle konventionellen Therapieformen versagt hatten und die sich im vollklinischen Endstadium der Aids-Erkrankung befanden. Allen Patienten wurden zunächst täglich intramuskuläre Dosen von jeweils 30 µg Glykopeptidlösung verabreicht, mit anschließender kombinierter parentaraler und oraler Verabreichung sowohl der Glykopeptidlösung einerseits als auch der Dragees andererseits, alternierend mit oraler Verabreichung von 300 mg Polyerger-Dragees. Diese Behandlung wurde dann in eine Langzeitbehandlung mit parentaraler Anwendung alle 48 Stunden und jeweils 3 x 100 mg oral zu nehmender Dragees an den dazwischen liegenden Tagen fortgesetzt. Alle Patienten befolgten eine halbvegetarische Diät.

Während der Behandlung wurden fortlaufend Serumparameter untersucht (BUN, Transaminasen, Phosphatasen, Creatinin, LDH, Immunoglubolinen, Elektrophorese, Virusserologie, T-Zellen, RBC, BBC und Schilling-Formel).

Die Bestimmung der T-Zellen, der T-Helfer und der T-Unterdrücker-Zellen (cytotoxische Zellen) erfolgte mittels monoklonaler Antikörper (lev. 4, lev. 3, lev. 2). Die prozentuale Anzahl der lev. 4, lev, 3 und lev. 2 bindender einkerniger Zellen wurde mittels Immunofluoreszenzanalyse (FACS) bestimmt.

Als Ergebnis zeigten sich sieben Vollremissionen. Ein Patient erkrankte an einem gallopierenden Kaposisarkoma und starb 28 Tage nach Therapiebeginn. Zwei Patienten, deren Krankheit bei Beginn der Therapie bereits am weitesten fortgeschritten war, sprachen auf die Behandlung nicht an und starben in erwartungsgemäßer Zeit. Die sieben Patienten mit Vollremission befinden sich auf 10 Monate nach Therapiebeginn noch in der Vollremissionsphase, wobei die orale Behandlung mit Polyerga-Dragees in der oben angegebenen Form weiter fortgesetzt worden ist.

Die durchgeführten klinischen Untersuche zeigen, daß sich Aids-Erkrankungen, die bislang in keiner weise therapiert werden konnten, durch die erfindungsgemäß vorgeschlagene Verwendung von physiologisch aktiven Polypeptiden bzw. Glykopeptiden günstig beeinflussen lassen. Bei allen Patienten, die noch nicht das Stadium III des Syndroms, d.h. die Entwicklung neoplastischer Prozesse, erreicht hatten, ergab sich eine rasche Remission der Symptome und der serologischen Abnormalitäten. Dies gibt erheblich Hoffnung für die weiteren Fortschritte in der Aidstherapie.

Die in der vorstehenden Beschreibung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

**Patentansprüche**

1. Verwendung eines physiologisch aktiven Polypeptids, hergestellt durch vollständige enzymatische Proteolyse reticulo-endothelialreicher Organe von Säugetieren bei einer Temperatur von nicht mehr als 45° C, zur Herrichtung einer Arzneimittelspezialität zur Aids-Therapie.

2. Verwendung nach Anspruch 1, dadurch gekennzeichnet, daß im Anschluß an die vollständige enzymatische Proteolyse das Reaktionsgemisch von den unlöslichen Bestandteilen getrennt und daraufhin das Polypeptid abgetrennt wird.

3. Verwendung nach Anspruch 1, dadurch gekennzeichnet, daß im Anschluß an die vollständige enzymatische Proteolyse die Feststoffe mechanisch abgetrennt und die Proteolyse-Lösung eingeengt und lyophilisiert wird.

4. Verwendung nach einem der vorangehenden Ansprüche, dadurch gekennzeichnet, daß die Proteolyse mit Pepsin, Trypsin oder Papain erfolgt.

**Claims**

1. Use of physiologically active polypeptide, produced by complete enzymatic proteolysis of mammalian organs high in reticuloendothelial at a temperature of not more than 45°C for the preparation of a medication specialty for AIDS therapy.

2. Use according to claim 1, characterized in that, following the complete enzymatic proteolysis, the reaction mixture is separated from the insoluble constituents and the polypeptide is subsequently separated.

3. Use according to claim 1, characterized in that, following of complete enzymatic proteolysis, the solids are mechanically separated and the proteolysis solution is concentrated and lyophilized.

4. Use according to one of the preceding claims, characterized in that the proteolysis ensues with pepsin, trypsin or papain.

**Revendications**

1. Utilisation d'un polypeptide physiologiquement actif fabriqué par protéolyse enzymatique complète d'organes du système réticulo-endothélial de mammifères à une température ne dépassant pas 45 degrés C, pour la préparation d'une composition médicamenteuse pour la thérapie du SIDA.

2. Utilisation selon la revendication 1, caractérisée par le fait que, à la suite de la protéolyse enzymatique complète, le mélange réactif des parties subsistantes insolubles est séparé et après quoi le polypeptide est séparé.

3. Utilisation selon la revendication 1, caractérisée par le fait que, à la suite de la protéolyse enzymatique complète, la matière solide est séparée mécaniquement et la solution de protéolyse est concentrée et lyophilisée.

4. Utilisation selon l'une des revendications précédentes, caractérisée par le fait que la protéolyse s'effectue avec de la pepsine, trypsine ou papaïne.